# DEMANDE DE BREVET EUROPEEN

(11) **EP 0 642 807 A2**
(43) Date de publication de la demande: **15.03.1995**
(21) Numéro de dépôt: 94113702.8
(22) Date de dépôt: 01.09.1994
(51) Int. Cl.: A61N 1/16, A61N 2/00

(54) **Dispositif de protection contre les champs magnétiques**

(30) Priorité: 09.09.1993 IT VI930144
(71) Demandeur: Schirato, Bruno, I-36061 Bassano del Grappa (Vicenza) (IT)
(72) Inventeur: Schirato, Bruno, I-36061 Bassano del Grappa (Vicenza) (IT)
(74) Mandataire: Bettello, Pietro, Dott. Ing.

(57) **Abrégé**

Le dispositif comprend un support (1) à profil substantiellement rectangulaire, dans lequel est incorporée une série de bandes magnétiques (2) disposées parallèlement aux petits côtés du support précité. Au niveau de son bord périphérique, ce support comporte un élément auto-oscillant constitué par un faisceau de fils en une matière électriquement conductrice, ledit faisceau, alimenté par le champ magnétique terrestre, assure la déviation de celui-ci en vue d'éliminer les influx géopathogènes autour du support sus-mentionné.

## Description

La présente invention a pour objet un dispositif propre à assurer la protection du corps humain à l'encontre de l'influence des champs électromagnétiques externes.

La présence de ces influx sur le corps humain a été pour la première fois avancée par l'Allemand HARTMANN, pour lequel le champ magnétique terrestre recouvre la surface de la planète à la manière d'un réticule. Selon HARTMANN, le réticule dans lequel la terre est subdivisée est composé de mailles d'environ 2 x 2,5 m., tissées avec un fil imaginaire dont l'épaisseur est d'environ 21 cm.

Au croisement des mailles de ce réticule se créent des "noeuds" qui représenteraient la concentration des énergies négatives. Au niveau des zones d'altération du champ magnétique terrestre, à ces énergies viennent s'ajouter les perturbations bien connues provoquées par le fonctionnement des appareils électriques, notamment des téléviseurs et des ordinateurs.

L'état de la technique (voir le Modèle d'utilité italien n° 217 662 et EP-A- 332 086) démontre qu'on a proposé d'utiliser, à des fins thérapeutiques, des enveloppes de revêtement, par exemple pour des matelas, comportant des bandes magnétiques disposées de différentes façons.

Dans le but d'obtenir une armature efficace à l'encontre des perturbations magnétiques ci-dessus mentionnées, la demande de brevet italien N° VI 92A000118 du même Demandeur prévoit l'utilisation d'une série de bandes magnétiques disposées parallèlement les unes aux autres à l'intérieur d'un support qui affecte avantageusement la forme d'une couverture pour lits, pour divans et/ou pour fauteuils.

Les essais qui ont été effectués ont permis de vérifier qu'un tel dispositif est en mesure de neutraliser de manière très efficace l'influence néfaste des champs magnétiques au niveau des zones de perturbations sus-indiquées.

La présente invention vise essentiellement à améliorer le dispositif ci-dessus décrit, et elle a pour objet le dispositif nouveau qui est défini à la revendication 1.

En fait, l'invention prévoit que le long du bord périphérique du support est prévu un faisceau de fils en une matière électriquement conductrice, lequel faisceau présente deux courtes interruptions à proximité de la médiane de l'un des côtés du support, ces interruptions étant disposées symétriquement de part et d'autre de ladite médiane.

Les tests qui ont été réalisés ont permis de constater une amélioration surprenante en ce qui concerne le neutralisation des champs électromagnétiques au niveau des zones géopathogènes.

En pratique, le dispositif suivant l'invention permet d'obtenir autour du support une cellule à l'intérieur de laquelle la valeur du champ magnétique est pratiquement nulle, ce qui assure l'annulation des influences néfastes au niveau des zones géopathogènes.

Sur le plan pratique, le dispositif sus-mentionné fonctionne à la manière d'un oscillateur électromagnétique qui est ainsi propre à amortir, jusqu'à presque les annuler, les radiations géopathogènes qui, bien qu'à des valeurs de potentiel très basses, parviennent à la longue à irriter l'organisme exposé pendant de longues périodes de temps à leur action nuisible.

Dans ses revendications dépendantes, l'invention vise en outre certaines formes particulières de réalisation du dispositif précédemment décrit, ces formes de réalisation étant propres à rendre son utilisation spécialement efficace.

Le dessin annexé, donné à titre d'exemple, permettra de mieux comprendre l'invention, les caractéristiques qu'elle présente et les avantages qu'elle est susceptible de procurer :
Fig. 1 est une vue en plan par dessus du dispositif suivant l'invention.
Fig. 2 est une coupe transversale suivant le plan indiqué en II-II en fig. 1, représentant l'oscillateur prévu dans le dispositif suivant fig. 1.
Fig. 3 montre une bande magnétique incorporée à celui-ci.
Fig. 4 représente schématiquement l'allure des lignes de champ magnétique telles que déviées par la présence du dispositif suivant l'invention.

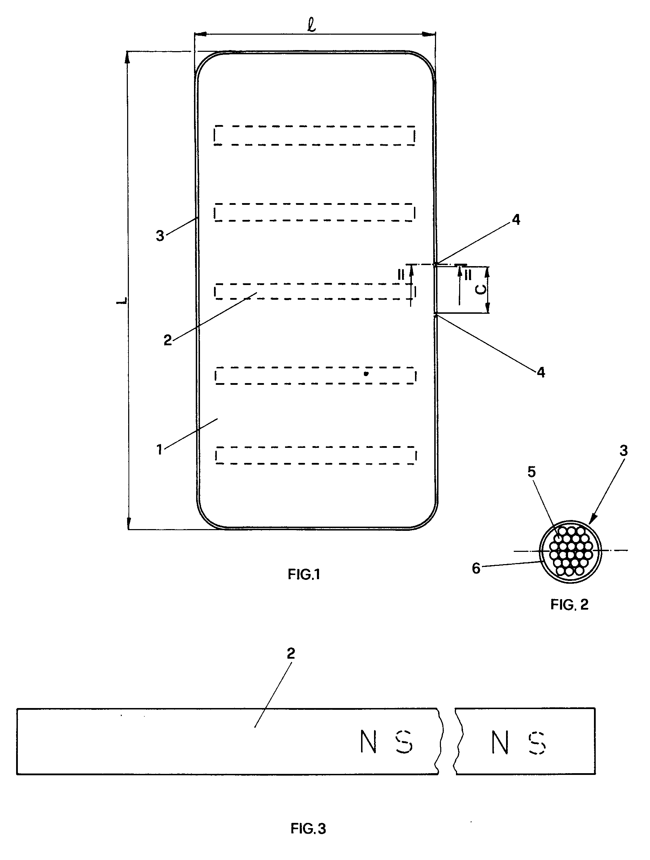

En fig. 1, on peut voir que ce dispositif est constitué par un support 1 à profil substantiellement rectangulaire, dans lequel est incorporée une série de bandes magnétiques 2, orientées parallèlement aux petits côtés dudit support.

Au niveau du bord périphérique de celui-ci est prévu un faisceau 3 de fils en une matière électriquement conductrice, lequel faisceau comporte deux interruptions 4 à proximité de la médiane de l'un des grands côtés du support ; ces interruptions 4 sont disposées symétriquement de part et d'autre de la médiane précitée.

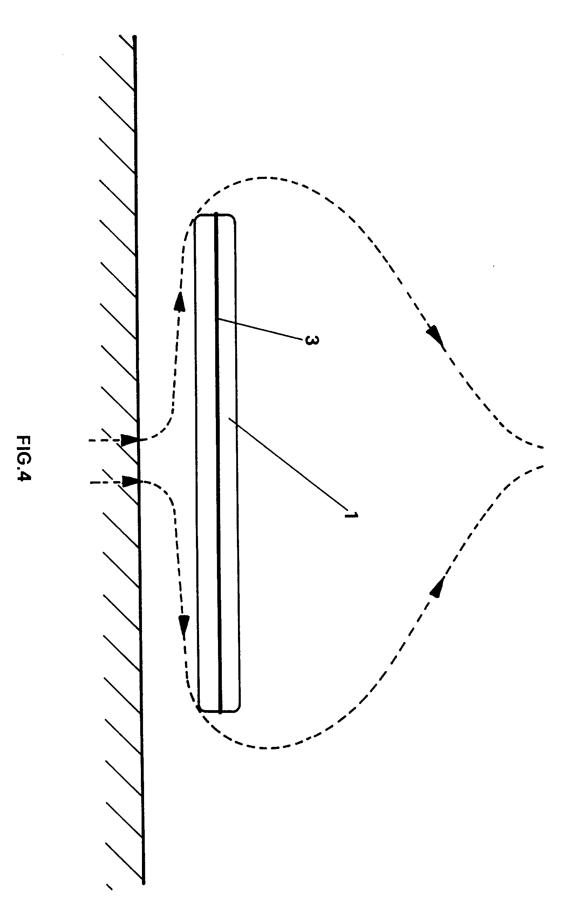

A la manière montrée en fig. 2, le faisceau 3 comporte douze fils en cuivre qui présentent préférablement un diamètre de 0,18 mm et qui sont diamétralement opposés à douze autres fils, également réalisés en cuivre, préférablement de section 0,20 mm.

Effectivement, c'est cette disposition structurelle qui a permis d'obtenir les meilleurs résultats. Les dimensions optimales du support sont les suivantes :
- Longueur (L) : 190 à 200 cm
- Largeur (l) : 80 à 180 cm
- Distance (C) séparant les interruptions : 20 cm
- Largeur de ces interruptions : 0,2 à 0,4 cm.

On peut constater que les dimensions ci-dessus indiquées, tout spécialement en ce qui concerne la longueur et la largeur, permettent une utilisation aisée du dispositif suivant l'invention sur un lit, sur un divan ou même à la manière d'un tapis.

Fig. 4 montre que par suite de l'action du dispositif suivant l'invention, les radiations géopathogènes sont en fait annulées au niveau d'une vaste zone située au-dessus du support 1. En pratique, les lignes de champ magnétique sont contraintes de diverger au-dessus du support et de ne converger que bien au-dessus de celui-ci, en engendrant une zone pratiquement dépourvue de toute influence géopathogène.

En fig. 2, on peut voir en outre que le faisceau de fils 5 est complètement entouré par une couche 6 en un matériau isolant, ceci aussi bien en vue d'assurer une isolation électrique que pour renforcer structurellement tout le système auto-oscillant.

L'exposé qui précède rend évident le fait que le dispositif suivant l'invention est en mesure d'exercer une action extrêmement simple et efficace pour l'obtention des buts qui étaient fixés. On notera en particulier que le fonctionnement de l'oscillateur ne nécessite aucune source auxiliaire d'énergie, ce qui constitue un avantage notable pour la simplicité du dispositif.

En outre, on observera que les bandes magnétiques 2 présentent préférablement deux polarités opposées sur chacune des deux faces du support 1.

## Revendications

1. Dispositif pour la protection du corps humain à l'encontre de l'influence des champs électromagnétiques externes, du genre comprenant un support (1) dans lequel est incorporé une série de bandes magnétiques (2), caractérisé en ce que les bandes (2) sont disposées parallèlement aux petits côtés du profil rectangulaire du support et en ce qu'il est prévu le long du bord périphérique de celui-ci un faisceau (3) de fils en une matière électriquement conductrice, lequel faisceau comporte deux courtes interruptions (4) à proximité de la médiane d'un des grands côtés du support, ces interruptions étant disposées symétriquement par rapport à cette médiane.

2. Dispositif suivant la revendication 1, caractérisé en ce que le faisceau (3) comprend douze fils de cuivre présentant un diamètre de 0,18 mm auxquels sont diamétralement opposés douze autres fils de cuivre présentant un diamètre de 0,20 mm.

3. Dispositif suivant l'une quelconque des revendications 1 et 2, caractérisé en ce que la longueur des grands côtés du support (1) varie de 190 à 200 cm, celle des petits côtés de 80 à 180 cm, tandis que la longueur de la partie comprise entre les deux interruptions (4) est de 20 cm et que la largeur de ces interruptions varie entre 0,2 à 0,4 cm.

4. Dispositif selon l'une quelconque des revendications précédentes, caractérisé en ce que les bandes magnétiques (2) présentent deux polarités opposées au niveau de chacune des deux faces du support (1).
